# EUROPÄISCHE PATENTANMELDUNG

(11) **EP 3 960 102 A1**
(43) Veröffentlichungstag der Anmeldung: **02.03.2022**
(21) Anmeldenummer: 20193373.6
(22) Anmeldetag: 28.08.2020
(51) Int. Cl.: A61B 17/34, A61M 25/00

(54) **TRANSSEPTALER KATHETER**

(71) Anmelder: atHeart Medical AG, 6340 Baar (CH)
(72) Erfinder: BERNHARD, Jérôme, 8003 Zürich (CH); CHRISTEN, Lukas, 6004 Luzern (CH); MELLMANN, Andreas, 6315 Oberägeri (CH)
(74) Vertreter: Grünecker Patent- und Rechtsanwälte PartG mbB

(57) **Zusammenfassung**

Die vorliegende Erfindung betrifft einen zum radialen Aufdehnen des Septums und/oder Occluders geeignet ausgebildeten transseptalen Katheter mit einem einem distalen Ende des Katheters zugeordneten Konusbereich (22), der die radiale Außenabmessung des Katheters gegenüber dem distalen Ende vergrößert, und einer zu dem Konusbereich (22) proximal benachbarten Dilatationseinrichtung (20).

## Beschreibung

Die Technik der transseptalen Punktion (TP) wurde in den späten 1950er Jahren von Ross et al. als diagnostisches Instrument zur richtigen Auswahl von Patienten für eine Herzklappenoperation in die klinische Praxis eingeführt. Im Laufe der Jahre ist der Einsatz von TP für Diagnosezwecke zurückgegangen, es wurde aber eine Reihe neuer interventioneller Verfahren entwickelt, die TP einsetzen. Die derzeit häufigsten Indikationen sind elektrophysiologische Ablationen am linken Herzen, perkutane Mitralklappenreparatur bzw. -implantation, Verschluss des Foramen ovale, Verschluss des Anhangsgebilde des linken Vorhofs, Reparatur von paraprothetischen Klappenlecks bzw. Positionierung der linksventrikulären Unterstützungsvorrichtung.

Die zunehmende Zahl der interventionellen Eingriffe sowie die Verwendung von Schleusen mit großem Durchmesser und komplexen Geräte haben zu verbesserter Technik und Ausrüstung geführt. TP ist jedoch immer noch eine technische Herausforderung. Daher ist eine spezielle Schulung erforderlich, denn nur sachkundige Ärzte sollten TP durchführen. Da die anatomischen Gegebenheiten unter Durchleuchtung nicht sichtbar gemacht werden können, ist die Verwendung der Echokardiographie unerlässlich geworden, um die Sicherheit zu erhöhen und eine genauere Bestimmung der Lage des Durchstosspunktes innerhalb der Vorhofscheidewand zu ermöglichen, vgl. Guido Russo et al. "Transseptal Puncture: A Step-by Step Procedural Guide", in Cardiac Interventions Today, Mai/Juni 2019, Vol. 13, No. 3, S 22 ff.

Die US2017/0105761A1 offenbart ein Verfahren zum transvaskulären Einführen eines Führungsdrahtes in den rechten Vorhof eines Patienten. Dazu wird eine Vorrichtung offenbart, die einen Katheter und eine in einem Lumen des Katheters angeordnete Hohlnadel umfasst, wobei die Hohlnadel einen proximalen Bereich und einen distalen Bereich, der flexibler als der proximale Bereich ist, umfasst. Die Vorrichtung wird in das rechte Atrium eingeführt, indem die Nadel über den Führungsdraht geführt wird. Nach dem Einführen der Vorrichtung in das rechte Atrium wird der Führungsdraht teilweise in die Nadel zurückgezogen, während ein distales Ende des Führungsdrahts in der Nadel und in einem Körper des Patienten verbleibt. Anschließend wird in das intraaterielles Septum mit der Nadel ein Loch punktiert.

Abhängig von der in dem linken Vorhof vorzunehmenden Maßnahme muss ein Durchgang durch das Septum mitunter vergrößert werden, um beispielsweise eine Schleuse durch das Septum hindurch zu verlegen, welche dem Einführen weiterer Instrumente oder Prothesen dient. Mitunter muss der Katheter auch durch einen Occluder oder einen chirurgisch eingebrachten Patch hindurch verlegt werden, der eine Öffnung in dem Septum durchsetzt.

Im Hinblick auf diese Anforderungen will die vorliegende Erfindung einen verbesserten transseptalen Katheter angeben.

Hierzu schlägt die vorliegende Erfindung einen transseptalen Katheter mit den Merkmalen von Anspruch 1 vor. Dieser transseptale Katheter hat im Bereich seines distalen Endes einen Konusbereich. Dieser Konusbereich erweitert die Außenabmessung des distalen Endes des Katheters. Benachbart zu diesem Konusbereich und proximal hierzu ist eine Dilatationseinrichtung vorgesehen.

So kann der transseptale Katheter mit seinem distalen Ende zunächst in eine relativ kleine Punktionsöffnung des Septums eingebracht werden. Beim fortschreitenden Hindurchführen des Katheters weitet der Konusbereich das Septum bzw. einen Occluder radial so weit auf, dass die Dilatationseinrichtung in die Öffnung des Septums gelangen kann. In dieser Lage wird die Dilatationseinrichtung aktiviert, um die Punktionsöffnung innerhalb des Septums oder den Occluder radial zusätzlich aufzuweiten.

Im Rahmen der Beschreibung der vorliegenden Erfindung wird auf Außenabmessungen des Katheters abgestellt. Hierbei handelt es sich um die radiale Erstreckung des Katheters. Damit soll indes nicht zum Ausdruck gebracht werden, dass der Katheter zwangsläufig eine kreisrunde Querschnittsform hat. Eine solche ist allerdings zu bevorzugen.

Gemäß einer ersten Variante kann der Katheter ein zum Durchstechen des Septums bzw. des Occluders angepasst ausgebildetes distales Ende haben, das an einem den Konusbereich ausbildenden und die Dilatationseinrichtung haltenden Katheterkörper ausgebildet ist. Bei dieser Variante ist der Katheterkörper durch ein einheitliches Gebilde ausgeformt, welches sich von dem distalen zu dem proximalen Ende erstreckt und das zum Durchstechen angepasst ausgebildete distale Ende hat. Dieses Ende ist üblicherweise spitz zulaufend nach Art einer Nadel ausgeformt. Der entsprechende Katheterkörper hat üblicherweise zwei Lumen, nämlich ein erstes Lumen, welches sich von dem proximalen bis zu dem distalen Ende erstreckt und sich beispielsweise zum Durchleiten eines Führungsdrahtes eignet. Der Führungsdraht kann nach dem Punktieren und Aufweiten des Septums durch die Dilatationseinrichtung bis hin zu der gewünschten Position linksseitig des Septums geschoben und zum Einführen von weiteren Instrumenten oder Prothesen, z.B. einer Herzklappe in den linken Vorhof genutzt werden. Die hier diskutierte Ausführungsform ist üblicherweise zusammen mit einer Schleuse vorgesehen, die beim Einbringen der Variante verhindert, dass das distale Ende des Katheterkörpers auf dem Weg in das Herz das zum Einführen benutzte Gefäß verletzt.

Die Schleuse kann an ihrem vorderen Ende gekrümmt oder steuerbar sein, um die gewünschte Positionierung des transseptalen Katheters an dem Septum zu verbessern. Auch im Übrigen folgt die Schleuse in der Regel den üblichen und an sich bekannten Vorgaben.

Bei einer atraumatischen Ausgestaltung ist das zum Durchstechen des Septums bzw. eines Occluders angepasst ausgebildete Ende an einem Punktionskörper vorgesehen. Dieser Punktionskörper ist in einem Dilatationskörper relativ beweglich und bildet üblicherweise das erste Lumen zum Hindurchführen des Führungsdrahtes aus. Als Dilatationskörper in diesem Sinne wird ein Körper verstanden, der den Konusbereich ausbildet und die Dilationseinrichtung hält. Bei der Benutzung dieser atraumatischen Variante wird zunächst der Dilatationskörper bei zurückgezogenem Punktionskörper bis zu dem Septum bzw. dem Occluder geführt. Erst dann wird der Punktionskörper in Längsrichtung relativ zu dem Dilatationskörper verschoben, um das Septum bzw. den Occluder mit dem distalen Ende des Punktionskörpers zu punktieren. Danach wird der Dilatationskörper vorgeschoben, üblicherweise relativ zu dem Punktionskörper, um das Septum bzw. den Occluder über den Konusbereich aufzuweiten und die Dilatationseinrichtung auf Höhe des Septums zu positionieren. Danach wird die Dilatationseinrichtung aktiviert, um das Septum bzw. den Occluder radial aufzuweiten. Bei diesem Vorgehen bleibt üblicherweise der Punktionskörper nach dem Durchstechen und beim Einbringen des Dilatationskörpers ortsfest und führt die Vorschubbewegung des Dilatationskörpers.

Gemäß einer bevorzugten Weiterbildung der vorliegenden Erfindung ist die Dilatationseinrichtung durch eine mechanische Spreizeinrichtung gebildet. Diese Spreizeinrichtung hat zwei Halteabschnitte, die in Längsrichtung des Katheters aufeinander zu bewegbar sind. Zwischen diesen Halteabschnitten und in Längsrichtung des Katheters erstrecken sich Spreizsegmente, die mit den jeweiligen Halteabschnitten verbunden sind. Die Halteabschnitte können beispielsweise auf dem Außenumfang des Katheters montiert bzw. beweglich geführt sein. So kann beispielsweise einer der Halteabschnitte mit dem Außenumfang des Katheters fest verbunden sein, während ein anderer mit einem Rohr oder Schlauch zusammenwirkt, die über den Katheter geschoben werden kann und mit welcher die beiden Halteabschnitte aufeinander zubewegt werden können. Dies erfolgt nach angemessener Positionierung der Spreizeinrichtung im Bereich des Septums wie zuvor beschrieben. Durch das Aufeinanderzubewegen der Halteabschnitte werden die Spreizsegmente radial aufgeweitet. Die entsprechende mechanische Spreizeinrichtung kann beispielsweise durch eine Hülse gebildet sein, bei welcher durch Schlitzen des Hülsenmaterials zwischen den beiden Halteabschnitten die Spreizsegmente ausgeformt werden. Die Spreizeinrichtung kann aus Kunststoff oder Metall ausgebildet sein.

Das zuvor erwähnte erste Lumen, das im Folgenden auch als Katheterlumen bezeichnet wird, ist üblicherweise als von dem proximalen bis zu dem distalen Ende des transseptalen Katheters durchgehendes Katheterlumen ausgebildet. Die Dilatationseinrichtung kann als Ballon ausgeformt sein. In diesem Fall ist ein zweites Lumen zum Aktivieren des Ballons ausgebildet, das üblicherweise mit einem radial von dem transseptalen Katheter an seinem proximalen Ende abgehenden Anschluss, beispielsweise für Kochsalzlösung und/oder Kontrastmittel, kommuniziert.

Die Dilatationseinrichtung nach der vorliegenden Erfindung ist eine Einrichtung, deren radiale Abmessung im kollabierten Zustand üblicherweise nicht größer als die größte radiale Abmessung des Konusbereiches ist, so dass die kollabierte Dilatationseinrichtung in die durch den Konusbereich aufgeweitete Punktionsöffnung eingebracht und auf Höhe des Septums angeordnet werden kann. Im expandierten Zustand überragt die Dilatationseinrichtung radial den Konusbereich. Dabei kann die Dilatationseinrichtung im expandierten Zustand radial um den Faktor 2 bis 20 größer als die größte Abmessung des Konusbereiches sein.

Mit der vorliegenden Erfindung wird ein transseptaler Katheter vorgeschlagen, der das Septum bzw. den Occluder punktieren und nach der Punktion die durch das Punktieren geschaffene Öffnung radial aufweiten kann, wozu einerseits das Septum und/oder der Occluder mechanisch durch den Konusbereich und danach vorgegeben durch die Betätigung der Dilatationseinrichtung radial aufgeweitet wird. Als Dilatationseinrichtung kann ein Ballon verwendet werden. Die Dilatationseinrichtung wirkt dabei üblicherweise beim Aktivieren rein radial auf das Septum bzw. den Occluder. Die radiale Aufweitung unter axialem Einbringen des Konusbereiches sollte auf ein Minimum beschränkt werden. Dies gilt insbesondere dann, wenn der Durchtritt zwischen dem rechten und dem linken Vorhof durch einen Occluder zu erfolgen hat, der das Septum durchsetzt und verschließt. Das radiale Aufweiten eines Occluders allein durch einen Konusbereich bringt die Gefahr mit sich, dass die hierbei notwendige axiale Relativbewegung zwischen dem Katheter und dem Occluder diesen beschädigt oder aus seiner Lage verschiebt. Dieses Problem wird mit der Dilatationseinrichtung des erfindungsgemäßen Katheters vermieden.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Fig. 1: ein Ausführungsbeispiel einer transseptalen Katheteranordnung;
- Fig. 2: eine vergrößerte Darstellung des distalen Endes einer ersten Variante vor dem Aktivieren der Dilatationseinrichtung (Fig. 2a) und nach dem Aktivieren (Fig. 2b);
- Fig. 3: eine Variante des in Fig. 2 gezeigten Ausführungsbeispiels vor dem Vorschieben eines Punktionskörpers (Fig. 3a) und danach (Fig. 3b) und
- Fig. 4: ein Ausführungsbeispiel einer mechanischen Spreizeinrichtung.

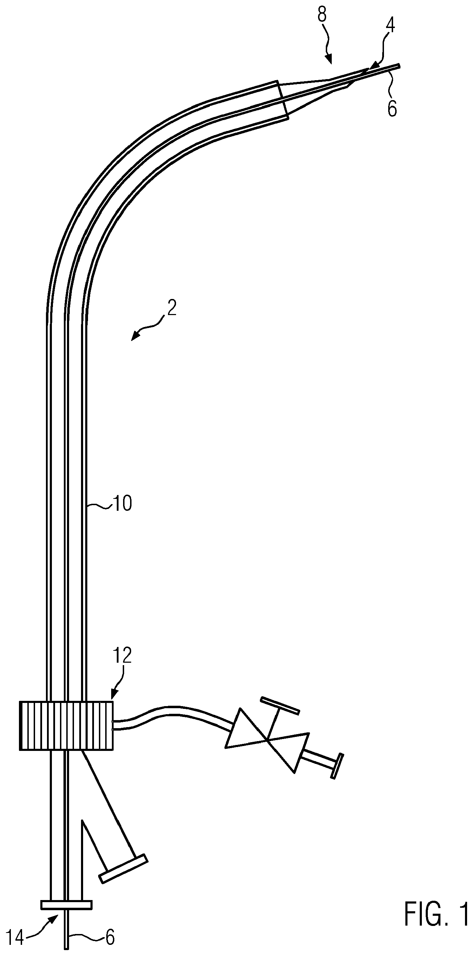

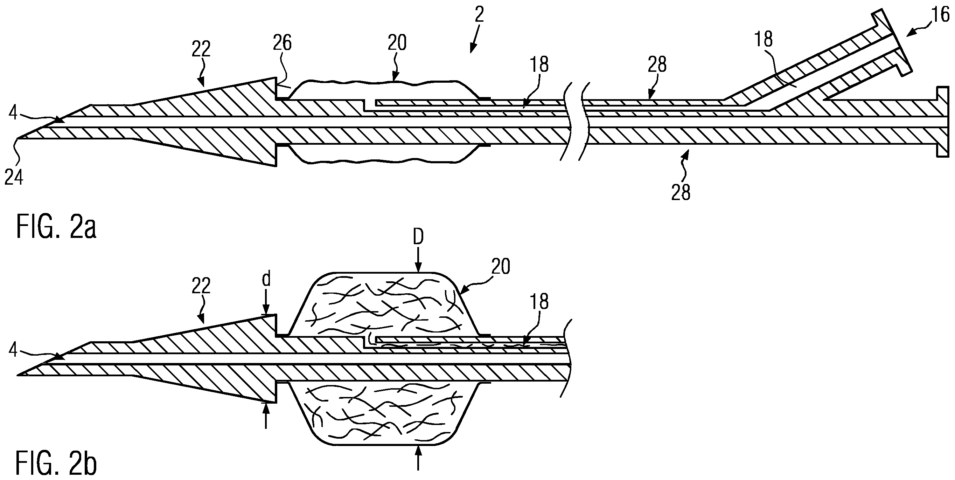

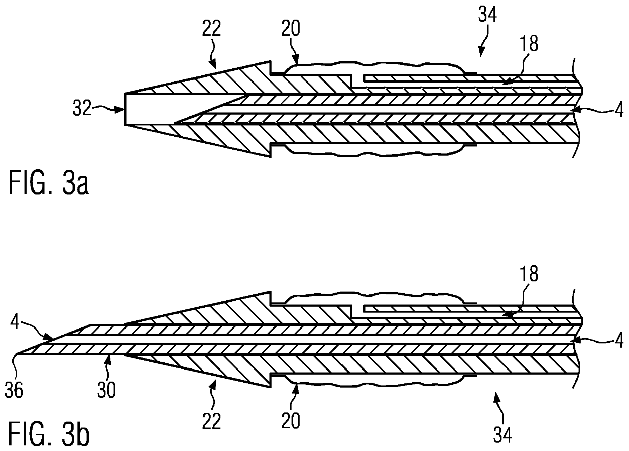

Die Fig. 1 zeigt eine Katheteranordnung mit einem mit Bezugszeichen 2 gekennzeichneten transseptalen Katheter, der in seinem ersten Lumen 4 einen Führungsdraht 6 führt, der in der Darstellung gemäß Fig. 1 ein distales Ende 8 des Katheters 2 überragt. Der Katheter 2 ist in einer Schleuse 10 aufgenommen und überragt ein proximales Ende 12 der Schleuse 10. Mit Bezugszeichen 14 ist ein proximales Ende des Katheters 2 gekennzeichnet. Dort ragt der Führungsdraht 6 aus dem Katheterlumen 4 heraus. Unmittelbar vor dem proximalen Ende 14 zweigt ein Anschluss ab, der mit einem zweiten, in den Fig. 2 und 3 mit Bezugszeichen 18 gekennzeichneten Lumen des Katheters 2 kommuniziert. Dieses zweite Lumen dient der Aktivierung eines Ballons 20, der als Ausführungsbeispiel einer Dilatationseinrichtung vorgesehen ist.

Dem Ballon 20 distal vorgelagert befindet sich ein Konusbereich 22, der distal absatzlos in den Außendurchmesser einer Spitze 24 übergeht, die zum distalen Ende 8 distal spitz zulaufend ausgeformt ist. Der Übergang zwischen dem Außendurchmesser der Spitze 24 und dem Konusbereich kann konkav gekrümmt sein. Wesentlich ist ein absatzfreier glatter Übergang zwischen den beiden Oberflächen der Spitze 24 einerseits und des Konusbereiches 22 andererseits. Das gegenüberliegende Ende des Konusbereiches 22 hat eine Ringfläche 26, die sich streng radial erstreckt. Unmittelbar benachbart zu dieser Ringfläche 26 und proximal hinter dem Konusbereich 22 befindet sich der Ballon 20.

Dieser ist in Fig. 2b in seinem expandierten Zustand gezeigt. Dabei wird üblicherweise Kochsalzlösung und/oder Kontrastmittel durch das zweite Lumen 18 in den Katheter 2 eingeleitet, wodurch der Ballon radial aufgeweitet wird. Die radiale Aufweitung kann bis zu einem Außendurchmesser D erfolgen, der üblicherweise um den Faktor 2 bis 20 größer als der größte radiale Außendurchmesser d des Konusbereiches 22 ist.

Während bei dem in Fig. 2 gezeigten Ausführungsbespiel das Katheterlumen 4 in einem mit Bezugszeichen 28 gekennzeichneten einheitlichen Katheterkörper durchgehend ausgeformt ist, wird dieses Katheterlumen 4 bei der Variante nach den Fig. 3a, 3b durch einen Punktionskörper 30 gebildet, der in einem Führungslumen 32 eines mit Bezugszeichen 34 gekennzeichneten Dilatationskörpers geführt ist, der vorliegend den Konusbereich 22 sowie das zweite Lumen 18 ausformt und den Ballon 20 hält. Das distale Ende dieses Dilatationskörpers 34 endet stumpf.

In Fig. 3a ist der Punktionskörper 30 gegenüber dem Dilatationskörper 34 zurückgezogen, so dass eine durch den Punktionskörper 30 ausgeformte Punktionsspitze 36 sich innerhalb des Dilatationskörpers 34 befindet. In Fig. 3b ist der Punktionskörper 30 vorgeschoben, so dass die Punktionsspitze 36 das distale Ende des Dilatationskörpers 34 überragt.

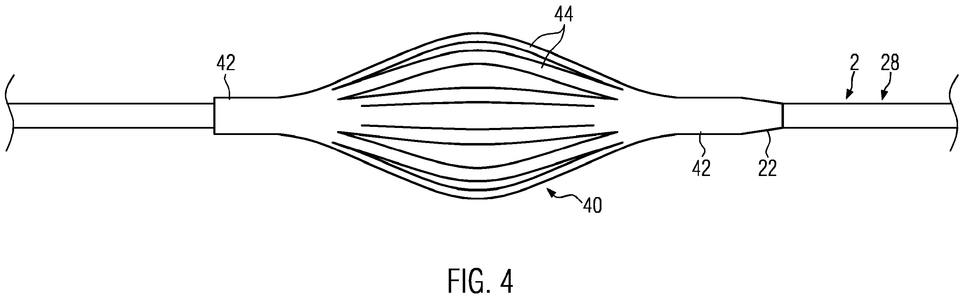

Die Figur 4 zeigt eine Spreizeinrichtung 40, die zwei in Längsrichtung des Katheters 2 beabstandet zueinander vorgesehene Halteabschnitte 42 aufweist, die bei dem gezeigten Ausführungsbeispiel als Hülsen um den Außenumfang des mit Bezugszeichen 28 gekennzeichneten Katheterkörpers ausgebildet sind. Dieser Katheterkörper 28 bildet - wie bei dem Ausführungsbeispiel nach Figur 2a, 2b - das Katheterlumen 4 aus.

Der in Figur 4 rechts gezeigte Halteabschnitt 42 ist fest mit dem Katheterkörper 28 verbunden. Der Außenumfang des Katheterkörpers 28 geht über eine Rampenfläche, die den Konusbereich 22 ausbildet, in den Außenumfang des Halteabschnitts 42 über. So ist hier eine gleichförmige Aufweitung des Radius bis auf den Außendurchmesser des Halteabschnitts 42 gegeben. Zwischen den beiden Halteabschnitten 42 befinden sich Spreizsegmente 44, die in Figur 4 bereits leicht radial durch Aufeinanderzuschieben der beiden Halteabschnitte 42 aufgeweitet sind. Die Spreizsegmente 44 können beispielsweise durch Längsschlitze eines zunächst einheitlichen Hülsenmaterials gebildet sein, welches endseitig die beiden Halteabschnitte 42 ausbildet. Der in Figur 4 linke Halteabschnitt 42 ist verschieblich auf dem Außenumfang des Katheterkörpers 28 gehalten. Durch einen weiteren Aktivierungskatheter, der über den Außenumfang des Katheterkörpers 28 geführt wird und der sich gegen die freie Stirnfläche des Halteabschnitts 42 anlegt, kann dieser linke Halteabschnitt 42 in Richtung auf den rechten Halteabschnitt 42 verschoben werden. Hierdurch weiten sich die einzelnen Spreizsegmente radial weiter auf.

Die Benutzung dieser Variante erfolgt wie die Benutzung der zuvor beschriebenen Varianten. Die Dilatationseinrichtung wirkt aktiviert, wenn sich das Septum in etwa auf mittlerer Höhe der Spreizsegmente 44 befindet.

### Bezugszeichenliste

- 2: Katheter
- 4: Katheterlumen
- 6: Führungsdraht
- 8: distales Ende
- 10: Schleuse
- 12: proximales Ende der Schleuse
- 14: proximales Ende des Katheters
- 16: Anschluss
- 18: zweites Lumen
- 20: Ballon
- 22: Konusbereich
- 24: Spitze
- 26: Ringfläche
- 28: Katheterkörper
- 30: Punktionskörper
- 32: Führungslumen
- 34: Dilatationskörper
- 36: Punktionsspitze
- 40: mechansiche Spreizeinrichtung
- 42: Halteabschnitt
- 44: Spreizsegment
- D: Außendurchmesser des aktivierten Ballons
- d: größter Außendurchmesser des Konusbereiches

## Patentansprüche

1. Transseptaler Katheter mit einem distalen Ende des Katheters zugeordneten Konusbereich (22), der die radiale Außenabmessung des Katheters gegenüber dem distalen Ende vergrößert, und einer zu dem Konusbereich (22) proximal benachbarten Dilatationseinrichtung (20; 40).

2. Transseptaler Katheter nach Anspruch 1 **gekennzeichnet durch** ein zum Durchstechen des Septums bzw. eines Occluders bzw. eines Patches angepasst ausgebildetes distales Ende (8), das an einem den Konusbereich (22) ausbildenden und die Dilatationseinrichtung (20; 40) haltenden Katheterkörper (28) ausgebildet ist.

3. Transseptaler Katheter nach Anspruch 1, **dadurch gekennzeichnet, dass** ein zum Durchstechen des Septums bzw. eines Occluders bzw. eines Patches angepasst ausgebildetes distales Ende an einem Punktionskörper (30) ausgebildet ist, der in einem den Konusbereich (22) ausbildenden und die Dilatationseinrichtung (20; 40) haltenden Dilatationskörper (34) relativ beweglich geführt ist.

4. Transseptaler Katheter nach einem der Ansprüche 1 bis 3, **gekennzeichnet durch** ein durchgehendes Katheterlumen (4) zur Führung eines Führungsdrahtes.

5. Transseptaler Katheter nach einem der vorherigen Ansprüche, **gekennzeichnet durch** eine Schleuse (10).

6. Transseptaler Katheter nach einem der vorherigen Ansprüche, **dadurch gekennzeichnet, dass** die Dilatationseinrichtung durch einen Ballon (20) gebildet ist.

7. Transseptaler Katheter nach einem der Ansprüche 1 bis 5, **dadurch gekennzeichnet, dass** die Dilatationseinrichtung durch eine mechanische Spreizeinrichtung (40) gebildet ist, die zwei in Längsrichtung des Katheters aufeinander zu bewegbare Halteabschnitte (42) umfasst, und an denen mehrere sich zwischen den Halteabschnitten (42) in Längsrichtung des Katheters (2) erstreckende Spreizsegmente (44) befestigt sind, die derart ausgebildet sind, dass sich deren radialer Abstand mit zunehmender Annäherung der Halteabschnitte (42) vergrößert.

8. Transseptaler Katheter nach einem der vorherigen Ansprüche, **dadurch gekennzeichnet, dass** die Dilatationseinrichtung (20; 40) im kollabierten Zustand radial zumindest nicht größer als die größte radiale Abmessung (d) des Konusbereiches (22) ist und im expandierten Zustand den Konusbereich (22) radial überragt.

9. Transseptaler Katheter nach einem der vorherigen Ansprüche, **dadurch gekennzeichnet, dass** die Dilatationseinrichtung (20; 40) im expandierten Zustand radial um den Faktor 2 bis 20 größer als der größte radiale Abmessung (d) des Konusbereiches (20) ist.

10. Verwendung eines Katheters, dessen radiale Außenabmessung gesteuert vergrößert werden kann zum Aufweiten eines Septrums, eines transseptalen Occluders oder eines Patches.
